# EUROPEAN PATENT APPLICATION

(11) **EP 2 072 039 A1**
(43) Date of publication of application: **24.06.2009**
(21) Application number: 07024961.0
(22) Date of filing: 21.12.2007
(51) Int. Cl.: A61K 8/64, A61Q 19/08, A61K 38/16

(54) **Use of a neurotoxic component of Clostridium botulinum toxin complex to reduce or prevent side effects**

(71) Applicant: Merz Pharma GmbH & Co. KGaA, 60318 Frankfurt (DE)
(72) Inventor: Haunold, Erich, Dr., 1140 Wien (AT); Marx, Matthias, Dr., 68119 Mannheim (DE)
(74) Representative: Ricker, Mathias

(57) **Abstract**

Use of a neurotoxic component of a *Clostridium* botulinum toxin complex for the facial cosmetic treatment of a human or animal susceptible to suffering from doll's face or frozen face, or the neurotoxic component of a *Clostridium* botulinum toxin complex for the treatment of a disease or disorder caused by or associated with hyperactive cholinergic innervation of muscles or exocrine glands in a human or animal susceptible to side effects associated with the treatment of said disease or disorder with a *Clostridium* botulinum toxin complex.

## Description

The present invention relates to the use of a neurotoxic component of a *Clostridium* botulinum toxin complex to reduce or prevent side effects in a human or animal which is often observed in humans or animals treated with a *Clostridium* botulinum toxin complex or a cosmetic composition containing a *Clostridium* botulinum toxin complex. One aspect of the present invention relates to the use of a neurotoxic component of a *Clostridium* botulinum toxin complex for the facial cosmetic treatment of a human or animal susceptible to suffering from doll's face or frozen face. A second aspect of the present invention relates to a neurotoxic component of a *Clostridium* botulinum toxin complex for the treatment of a disease or disorder caused by or associated with hyperactive cholinergic innervation of muscles or exocrine glands in a human or animal susceptible to side effects associated with the treatment of said disease or disorder with a *Clostridium* botulinum toxin complex.

A botulinum toxin complex is a bacterial toxin that prevents nerves from releasing acetylcholine, which is essential for the nerves to communicate with muscle cells. This toxin therefore prevents muscles from receiving nerve stimulation. Preventing nerve stimulation of muscles causes the muscles to become paralysed. Thus, a botulinum toxin complex is useful in treating conditions where excessive nerve stimulation to muscles is causing abnormal muscle activity. The toxin complex is also useful for preventing excessive sweating, as it prevents nerve signals from reaching the sweat glands in the same manner. The complex may also be used for the cosmetic treatment of lines and wrinkles, preferably facial lines and wrinkles. For the treatment of said muscle activity, excessive sweating or lines and wrinkles, the complex is injected into, or in the vicinity of, the affected muscle, skin or gland.

For example, it has been reported that the toxin complex is used since nearly two decades and has become one of the most aesthetic interventions carried out by physicians in the United States (Dermat Surg 2007; 33: S18-S25). The cosmetic use of the neurotoxic component of botulinum toxin complex is broadly described in WO 00/74703.

Hyperfunctional lines, such as glabellar frown lines, forehead lines, and crow's feet of humans are caused by contractions of the facial muscles that result in a pleating of the overlying skin. Activity of these muscles can lead to permanent wrinkles, which may give the affected person a negative facial expression, such as anger, anxiety, fear, or sadness. Botulinum toxin type A (BTXA) or, more precisely the botulinum toxin type A complex, has been used successfully for cosmetics, i.e. aesthetic purposes, in order to reduce or avoid these wrinkles. It is also possible to use other serotypes of botulinum toxin for cosmetics, such as serotype B, C, D, E, F and G.

The procedure for cosmetically treating said lines or wrinkles involves injecting the purified botulinum toxin complex in very tiny amounts into a targeted facial muscle. The resulting nerve blockade of that muscle causes a local immobilisation of muscle movement, which prevents "crinkling" and wrinkle lines from forming when the treated person frowns or squints (http://www.parentmap.com/may_06/0506_4a.htm).

It is further known to widely use botulinum toxin A (BTXA) as a drug in cosmetic dermatology, not only to treat focal hyperhidrosis but also hyperkinetic facial lines, platysma bands, decollete bands, and other skin features.

However, the spectrum of possible adverse effects of BTXA in cosmetic use is broad. The most common adverse effects are pain and hematoma. In the periocular region, lid and brow ptosis are important adverse effects. Adverse effects such as pain, hematoma, ecchymosis, and bruising may also occur in the upper and lower face and at extrafacial sites. Other possible adverse effects seen in other indications that the user of BTXA in cosmetic dermatology should be wary of include induction headaches and possible interaction with concomitant medications.

From the medical point of view, the major tools for preventing adverse effects from BTXA in the cosmetic treatment are knowledge and skill. Use of correct injection techniques is mandatory since most unwanted effects are caused by incorrect technique. Knowledge of the target structures, e.g. the facial and extrafacial muscles, allows physicians to select the optimal dose, time and technique. Of these, the most important for avoiding most unwanted adverse effects are the proper techniques of dilution, storage, and injection, as well as the careful exclusion of treated persons with any contraindications. Pain, hematoma, ecchymosis, and bruising can be prevented by cooling the skin before and after BTXA injection. Upper lid ptosis may be partly corrected using apraclonidine or phenylephrine eyedrops. If simple rules relating to the indications for and application of BTXA are followed, this is a safe and effective drug in cosmetic dermatology (Am J Dermatol 2005;6 (3): 141 - 50).

Besides the above mentioned adverse side effects in the cosmetic treatment of lines and wrinkles of the skin of a person with a botulinum toxin complex, also the adverse problem of the "doll's face" respectively the "frozen face" may arise. The terms "doll's face" and respectively the "frozen face" mean the mask-like or doll-like appearance of the face as a result of a treatment with a botulinum toxin complex, which has to be attributed to a significant suppression of the mimic of the face. Instead of giving the face a more relaxed and smoother appearance, the facial movement is immobilized e.g. static with the result of an emotionless face having an artificial, i.e. non-natural, look. Such look is often referred to in the literature and herein as a "doll's face" or a "frozen face". Accordingly, the treatment with a botulinum toxin complex has prevented the treated person from expressing emotions. The face can no longer express its full and subtle range of emotions. (http://www.wlbeauty.com/press_details.php?press_id=52; http://www.abc.net.au/science/k2/moments/s909485.htm).

These side effects may convey a severe psychological burden to persons which have been treated with a botulinum toxin complex in order to smoothen facial lines or wrinkles because the face no longer has a desired natural appearance respectively a desired natural e.g. dynamic mimic being able to express various emotions. However, until now, in the scientific literature, the problem of "doll's face" respectively the "frozen face" and problems associated therewith have not received the attention they deserve. Despite the methods to avoid or to ameliorate side effects mentioned above, the problem of the "doll's face" or the "frozen face" still exists.

Botulinum toxin complex is also used for the preparation of cosmetic compositions to successfully treat diseases or disorders in humans or animals caused by or associated with hyperactive cholinergic innervation of muscles or exocrine glands in a patient.

Such diseases or conditions involve dystonia of a muscle. Exemplarily mentioned are (1) cranial dystonia including blepharospasm, oromandibular dystonia of the jaw opening or jaw closing type, bruxism, Meige syndrome, lingual dystonia, apraxia of eyelid opening, (2) cervical dystonia including antecollis, retrocollis, laterocollis, torticollis (3) pharyngeal dystonia, (4) laryngeal dystonia including spasmodic dysphonia of the adductor type or of the abductor type, spasmodic dyspnea, (5) limb dystonia including arm dystonia such as task specific dystonias, including writer's cramp, musician's cramps or golfer's cramp, leg dystonia involving thigh adduction, thigh abduction, knee flexion, knee extension, ankle flexion, ankle extension or equinovarus deformity, foot dystonia involving striatal toe, toe flexion or toe extension, axial dystonia such as Pisa syndrome or belly dancer dystonia, segmental dystonia, hemidystonia or generalised dystonia, (6) dystonia in Lubag, (7) dystonia in corticobasal degeneration (8) tardive dystonia, (9) dystonia in spinocerebellar ataxia, (10) dystonia in Parkinson's disease, (11) dystonia in Huntington's disease, (12) dystonia in Hallervorden Spatz disease, (13) dopa-induced dyskinesias/dopa-induced dystonia, (14) tardive dyskinesias/tardive dystonia, (15) paroxysmal dyskinesias/dystonias (kinesiogenic, non-kinesiogenic, action-induced); or involves a clinical pattern selected from the group consisting of torticollis, laterocollis, retrocollis, anterocollis, flexed elbow, pronated forearm, flexed wrist, thumb-in-palm or clenched fist.

Such diseases or conditions may also involve spasticity of a muscle. In general, the spasticity is or is associated with (1) a spastic condition in encephalitis and myelitis relating to (a) autoimmune processes including respect to multiple sclerosis, transverse myelitis, Devic syndrome, (b) viral infections, (c) bacterial infections, (d) parasitic infections or (e) fungal infections, (2) hereditary spastic paraparesis, (3) postapoplectic syndrome resulting from hemispheric infarction, brainstem infarction or, myelon infarction, (4) a central nervous system trauma involving e.g. a hemispheric lesion, a brainstem lesion, a myelon lesion, (5) a central nervous system hemorrhage such as an intracerebral hemorrhage, a subarachnoidal hemorrhage, a subdural hemorrhage or an intraspinal hemorrhage, or (6) a neoplasia, e.g. a hemispheric tumor, a brainstem tumors or a myelon tumor or (7) post-stroke spasticity, or (8) spasticity caused by cerebral palsy.

It has already been reported that patients suffer from adverse side effects after the therapeutical treatment of the above mentioned diseases and disorders with a cosmetic composition containing a botulinum toxin complex (http://www.netdoctor.co.uk/medicines/100000363.html).

Reported general side effects include pain, soreness or bruising at the injection site, misplaced injections may paralyze nearby muscles and excessive doses may paralyze muscles that are not near the injection site, fever (pyrexia), flu-like symptoms, difficulty or pain when swallowing (dysphagia), rash or itching.

Specific side effects in the treatment of blepharospasm / hemifacial spasm have been reported to include easily bruising of the soft eyelid tissues, drooping of the upper eyelid, sensitivity of the eyes to light, eye irritation, dry eyes, watering eyes, facial swelling, difficulty closing the eyelid, inflammation of the front of the eye (cornea).
Specific side effects in the treatment of cervical dystonia patients have been reported to include dizziness, increased rigidity, stiffness or soreness of muscles, numbness, weakness, drowsiness, nausea, general feeling of being unwell/malaise.

Specific side effects in the treatment of cerebral palsy patients have been reported to include viral or ear infections, urinary incontinence, sleepiness, general feeling of being unwell/malaise, rash, tingling.

Specific side effects in the treatment of stroke patients have been reported to include arm pain, increased rigidity of muscles, general feeling of being unwell/malaise.

Specific side effects in the treatment of hyper hydrosis have been reported to include increased sweating from areas not treated, hot flushes.

The problem to be solved by the present invention was to reduce or prevent the side effects which may occur in the cosmetic facial treatment of facial lines and facial wrinkles and to maintain the natural e.g. dynamic mimic of the face in such a cosmetic treatment, or to reduce or prevent the side effects which may occur in the therapeutic treatment of a disease or disorder caused by or associated with hyperactive cholinergic innervation of muscles or exocrine glands in a human or animal associated with a botulinum toxin complex or a cosmetic composition containing said complex.

The problem was solved by using a neurotoxic component of a *Clostridium* botulinum toxin complex.

One aspect of the invention relates to the use of a neurotoxic component of a *Clostridium* botulinum toxin complex for the facial cosmetic treatment of a human or animal susceptible to suffering from doll's face or frozen face.

A human or animal is considered "susceptible" to suffering from e.g. doll's face, whenever the chance exists that this condition can occur, i.e. if the condition of doll's face already occurred before or the reasonable concern exists, that such a condition might occur. In particular the term "susceptible" encompasses the situation where a patient is afraid of developing doll's face in view of information (from e.g. media, friends, relatives, etc.) and/or earlier experience regarding side effects of a botulinum toxin treatment.

In one embodiment of this aspect of the invention, the human or animal have been treated with a *Clostridium* botulinum toxin complex and have suffered from or are concerned to suffer from doll's face or frozen face as a result of said treatment with the botulinum toxin complex.

In another embodiment, said facial cosmetic treatment is the smoothening of facial lines or facial wrinkles.

In yet another embodiment of the invention, the human or animal have been treated with a *Clostridium* botulinum toxin complex to smoothen facial lines and wrinkles of the skin.

In yet another embodiment, said facial cosmetic treatment is the smoothening of facial lines or facial wrinkles of the skin.

Preferably, the facial lines or facial wrinkles are selected from the group consisting of glabellar frown lines, frown lines, worry lines, marionette lines, horizontal forehead line, crow's feet, nose perioral fold, mental ceases, popply chin, and/or platysmal bands.

In the cosmetic treatment, the neurotoxic component is preferably intramuscularly or subcutaneously injected.

Preferably, the neurotoxic component is directly injected into the facial line or wrinkle to be smoothed, or is directly injected into, or in vicinity of, one or more facial muscles or muscles involved in the formation of the line or wrinkle of the skin to be smoothed.

The facial muscles comprise the following muscles: epicranius (occipitofrontalis, temporoparietalis), procerus, nasalis muscles (compressor nasalis and dilator nasalis), depressor septi nasi, orbicularis oculi, corrugator supercilii, depressor supercilii, auricular muscles (anterior, superior, posterior), orbicularis oris, depressor anguli oris, transversus menti, risorius, zygomaticus major, zygomaticus minor, levator labii superioris, levator labii superioris alaque nasi, depressor labii inferioris, levator anguli oris, modiolus, buccinator, mentalis, platysma.

The muscles involved in forming the facial lines and facial wrinkles mentioned above are preferably selected from the group consisting of corrugator supercillii, orbicularis oculi, procerus, venter frontalis of occipitofrontalis, orbital part of orbicularis oculi, nasalis, upper lip: orbicularis oris, lower lip: depressor anguli oris, mentalis and platysma.

Preferably, the neurotoxic component is directly injected into, or in vicinity of, one or two of said facial muscles or muscles involved in the formation of the facial line or facial wrinkle of the skin to be smoothed.

As already mentioned above, botulinum toxin may cause a local immobilisation of muscle movement. Accordingly, it may be assumed that the "doll's face" respectively "frozen face" arises from botulinum toxin complex which diffuses or migrates to areas around the injection point, and immobilizes further muscles not to be intended to be immobilized. It is known that the complex and the neurotoxin have comparable diffusion properties (Tang-Liu; R. Aoki et al., Toxicon 2003, 42, 461 - 469). Thus, in the use according to the invention, the neurotoxic component seems to have a lower diffusion or migration as compared to the complex resulting in an improved position stability as compared to the position stability of the botulinum toxin complex. This behaviour is surprising and could not be expected in view of the reported similar diffusion properties. Without wishing to be bound to this theory, it is believed that the local position stability of the neurotoxin, i.e. its reduced ability to diffuse or migrate and to immobilize other vicinal muscles as compared to the complex prevents the human or animal from "doll's face" respectively "frozen face".

A second aspect of the invention relates to a neurotoxic component of a *Clostridium* botulinum toxin complex for the treatment of a disease or disorder caused by or associated with hyperactive cholinergic innervation of muscles or exocrine glands in a human or animal susceptible to side effects associated with the treatment of said disease or disorder with a *Clostridium* botulinum toxin complex.

Side effects are preferably one or more of the following: pain, soreness or bruising at the injection site said, fever (pyrexia), flu-like symptoms, difficulty or pain when swallowing (dysphagia), rash or itching, bruising of soft eyelid tissues, drooping of the upper eyelid, sensitivity of the eyes to light, eye irritation, dry eyes, watering eyes, facial swelling, difficulty closing the eyelid, inflammation of the front of the eye (cornea), dizziness, increased rigidity, stiffness or soreness of muscles, numbness, weakness, drowsiness, nausea, general feeling of being unwell/malaise, viral or ear infections, urinary incontinence, sleepiness, tingling, arm pain, increased rigidity of muscles, increased sweating from areas not treated, hot flushes.

In one embodiment of this aspect of the invention, the human or animal have been treated with a *Clostridium* botulinum toxin complex and have suffered from one or more of the following: pain, soreness or bruising at the injection site said, fever (pyrexia), flu-like symptoms, difficulty or pain when swallowing (dysphagia), rash or itching, bruising of soft eyelid tissues, drooping of the upper eyelid, sensitivity of the eyes to light, eye irritation, dry eyes, watering eyes, facial swelling, difficulty closing the eyelid, inflammation of the front of the eye (cornea), dizziness, increased rigidity, stiffness or soreness of muscles, numbness, weakness, drowsiness, nausea, general feeling of being unwell/malaise, viral or ear infections, urinary incontinence, sleepiness, tingling, arm pain, increased rigidity of muscles, increased sweating from areas not treated, hot flushes.

For the treatment of a disease or disorder caused by or associated with hyperactive cholinergic innervation of muscles or exocrine glands, the neurotoxic component is intramuscularly or subcutaneously injected.

Preferably, the neurotoxic component is directly injected into, or in the vicinity of, the muscles or exocrine glands. Preferably, the neurotoxic component is directly injected into, or in the vicinity of one or two muscles or one or two exocrine glands.

The muscles are preferably selected from the group consisting of ipsilateral splenius, contralateral sternocleidomastoid, ipsilateral sternocleidomastoid, splenius capitis, scalene complex, levator scapulae, postvertebralis, ipsilateral trapezius, levator scapulae, bilateral splenius capitis, upper trapezius, deep postvertebralis, bilateral sternocleidomastoid, scalene complex, submental complex, brachioradialis, biceps brachialis, pronator quadratus pronator teres, flexor carpi radialis, flexor carpi ulnaris, flexor pollicis longus, adductor pollicis, flexor pollicis brevis/opponens, flexor digitorum superficialis, flexor digitorum profundus.

The exocrine gland is preferably selected from the group consisting of autonomic exocrine gland which generally is selected from the group consisting of sweat gland, tear gland, salivary gland and mucosal gland; or gland associated with a disease or condition selected from the group consisting of Frey syndrome, Crocodile Tears syndrome, axillar hyperhidrosis, palmar hyperhidrosis, plantar hyperhidrosis, hyperhidrosis of the head and neck, hyperhidrosis of the body, rhinorrhea, or relative hypersalivation in patients with stroke, Parkinson's disease or Amyotrophic Lateral Sclerosis.

Botulinum toxin is produced by the bacterium *Clostridium.* Botulinum toxins are released from lysed Clostridium cultures generally in the form of a sub-unit responsible for the toxic properties of the Botulinum toxin (the so-called "neurotoxic component") in association with other bacterial proteins, which together form a toxin complex - also designated "botulinum toxin complex". The botulinum toxin complex is metastable in nature, since its stability appears to depend on various factors such as e.g. salt concentration and/or pH. This complex usually contains additional, so-called "non-toxic" proteins, which we will refer to as "complexing proteins" or "bacterial proteins". The molecular weight of this complex may vary from about 300,000 to about 900,000 Da, i.e. from 300 kDa to about 900 kDa. The complexing proteins are, for example, various hemagglutinins. The proteins of this toxin complex are not toxic themselves but are believed to provide stability to the neurotoxic component and are responsible for oral toxicity in Botulinum intoxications. There are seven antigenically distinct serotypes of Botulinum toxin, namely Botulinum toxin A, B, C, D, E, F and G. Wherever the Botulinum toxin serotype A, B, C, D, E, F or G are mentioned, also known variants of the serotypes are encompassed, like serotypes A1, A2, A2, B1, B2, B3, C1, C2, C3, D1, D2, D3, E1, E2, E3, F1, F2, F3, or G1, G2, G3. As indicated in the introductory part of the application, there exist two commercially available formulation on the basis of the Botulinum toxin A protein complex, namely under the tradename BOTOX^{®} (Allergan Inc) and under the tradename DYSPORT^{®} (Ipsen Ltd)

The term "Botulinum toxin" as used throughout the present application, refer to the neurotoxic component devoid of any other Clostridial proteins, but also to the "Botulinum toxin complex". The term "Botulinum toxin" is used herein in cases when no discrimination between the toxin complex and the neurotoxic component is necessary or desired. "BoNT" or "NT" are common used abbreviations.

The "neurotoxic component" of the Botulinum toxin complex is initially formed as a single polypeptide chain, having in the case of serotype A a molecular weight of approximately 150 kDa. In other serotypes the neurotoxic component has been observed to vary between about 145 and about 170 kDa, depending on the bacterial source. In the case of serotype A, for example, proteolytic processing of the polypeptide results in an activated polypeptide in the form of a dichain polypeptide consisting of a heavy chain and a light chain, which are linked by a disulfide bond. In humans, the heavy chain mediates binding to pre-synaptic cholinergic nerve terminals and internalization of the toxin into the cell.

The term "neurotoxic component" also includes functional homologs found in the other serotypes of Clostridium botulinum. In a preferred embodiment of the present invention, the neurotoxic component is devoid of any other C. *botulinum* protein, preferably also devoid of RNA, which might potentially be associated with the neurotoxic component. The neurotoxic component may be the single chain precursor protein of approximately 150kDa or the proteolytically processed neurotoxic component, comprising the light chain (L_{c}) of approximately 50kDa and the heavy chain (H_{c}) of approximately 100kDa, which may be linked by one or more disulfide bonds (for a review see e.g. Simpson LL, Ann Rev Pharmacol Toxicol. 2004; 44:167-93). In humans, the heavy chain mediates binding to pre-synaptic cholinergic nerve terminals and internalization of the toxin into the cell. The light chain is believed to be responsible for the toxic effects, acting as zincendopeptidase and cleaving specific proteins responsible for membrane fusion (SNARE complex) (see e.g. Montecucco C., Shiavo G., Rosetto O: The mechanism of action of tetanus and Botulinum neurotoxins. Arch Toxicol. 1996; 18 (Suppl.): 342-354*)).*

The neurotoxic subunit of the Botulinum toxin complex is referred in this document as the "neurotoxic component" or the "neurotoxic component free of complexing proteins". A cosmetic composition comprising the neurotoxic component of Botulinum toxin type A in isolated form is commercially available in Germany from Merz Pharmaceuticals GmbH under the trademark Xeomin^{®}. The production of the neurotoxic component of Botulinum toxin type A and B are described, for example, in the international patent application WO 00/74703.

With regard to the composition and dosing of the medicament on the basis of Botulinum toxin, and in regard to the composition, dosing and frequency of administration of the medicament on the basis of the neurotoxic component of Botulinum toxin, reference is made to PCT/EP2007/005754.

In an preferred embodiment the Botulinum toxin is Botulinum toxin A. Serotype A is particularly preferred in the context of the present invention, because the duration of its therapeutic effect is superior when compared to the other serotypes. In the an even more preferred embodiment said Botulinum toxin is free of any complexing proteins (neurotoxic component), even more preferably it is the pure neurotoxic component serotype A. In addition thereto, modified as well as recombinant produced neurotoxic components of Botulinum toxins including the respective mutations, deletions, etc. are also within the scope of the present invention. With respect to suitable mutants, reference is made to WO 2006/027207 A1 and WO 2006/114308 A1, which are fully incorporated by reference herein. Furthermore, within the present invention, mixtures of various serotypes (in the form the neurotoxic component or recombinant form or both forms thereof, e.g. mixtures of Botulinum neurotoxins of types A and B) may be used. The present invention, however, also refers to neurotoxins which are chemically modified, e.g. by pegylation, glycosylation, sulfatation, phosphorylation or any other modification, in particular of one or more surface or solvent exposed amino acid(s).

In accordance with the teaching of the present invention it is particularly preferred that the medicament contains no proteins found in the botulinum toxin complex other than the neurotoxic component. The precursor of the neurotoxic component may be cleaved or uncleaved, however, it is preferred that the precursor has been cleaved into the heavy and the light chain. As pointed out elsewhere herein, the polypeptides may be of wild-type sequence or may be modified at one or more residues. Modification comprises chemical modification e.g. by glycosylation, acetylation, acylation or the like, which may be beneficial e.g. to the uptake or stability of the polypeptide. The polypeptide chain of the neurotoxic component may, however, alternatively or additionally be modified by addition, substitution or deletion of one or more amino acid residues.

The botulinum toxin, preferably the neurotoxic component referred to herein, may be part of a composition. This composition may contain the botulinum toxin/neurotoxic component as the sole active component or may contain additional pharmaceutically active components.

In a preferred embodiment of the present invention, the medicament comprises a serum albumin. In one embodiment, said serum albumin is a mammalian serum albumin, however, also comprised by this embodiment are albumin obtainable from other mammalians such as serum albumin derived from pig, bovine or primates as well as recombinantly produced albumins, as e.g. disclosed in EP 1 398 038. The albumin may be obtained by purification from the animal or by recombinant expression in a eukaryotic or prokaryotic cell. Alternatively, the albumin may be obtained by chemical synthesis. The aforementioned protein may be of wild-type sequence or may be modified at one or more residues. Modification comprises chemical modification e.g. by glycosylation, acetylation, acylation or the like, which may be beneficial e.g. to the uptake or stability of the polypeptide. The polypeptide chain of the albumin may, however, alternatively or additionally be modified by addition, substitution or deletion of one or more amino acid residues.

Preferably, said composition comprises the neurotoxic component of Botulinum toxin type A. Said composition is a reconstituted solution of the neurotoxic component of Botulinum toxin. Preferably, the composition further comprises sucrose or human serum albumin or both, still more preferably the ratio of human serum albumin to sucrose is about 1:5. In one embodiment, the composition is Xeomin^{®}. More preferably, said human serum albumin is recombinant human serum albumin. Alternatively, said composition is free of mammalian derived proteins such as human serum albumin. Any such solution may provide sufficient neurotoxin stability by replacing serum albumin with other non-proteinaceous stabilizers (infra).

The composition or cosmetic composition may contain additional pharmaceutically active components. "Cosmetic composition" is a formulation in which an active ingredient for use as a medicament or diagnostic is contained or comprised. Such cosmetic composition may be suitable for diagnostic or therapeutic administration (i.e. by intramuscular or subcutaneous injection) to a human patient. The cosmetic composition may be lyophilized or vacuum dried, reconstituted, or in solution. When reconstituted it is preferred that the reconstituted solution is prepared adding sterile physiological saline (0.9% NaCl).

The composition may comprise additional components such as a pH buffer, excipient, cryoprotectant, preservatives, stabilizer or any combination thereof.

The term "pH buffer" refers to a chemical substance being capable to adjust the pH value of a composition, solution and the like to a certain value or to a certain pH range. In one embodiment this pH range can be between pH 5 to pH 8, more preferably pH 7 to pH 8, even more preferably 7,2 to 7,6, and most preferably a pH of 7,4. The pH ranges given mentioned above are only typical examples and the actual pH may include any interval between the numerical values given above. Suitable buffers which are in accordance with the teaching of the present invention are e.g. sodium-phosphate buffer, sodium-acetate buffer, TRIS buffer or any buffer, which is suitable to buffer within the above pH-ranges.

The term "excipient" in this document refers to a substance present in a cosmetic composition other than the active pharmaceutical ingredient present in the cosmetic composition. An excipient can be a buffer, carrier, antiadherent, binder, disintegrant, filler, diluent, preservative, vehicle, cyclodextrin and/or bulking agent, such as albumin, gelatin, collagen and/or sodium chloride.

"Cryoprotectant" refers to excipients which result in the neurotoxic component in a reconstituted or aqueous solution cosmetic composition has greater than about 20%, 30%, 40%, 50%, 60%, 70%, 80%, 90%, and up to about 100% of the toxicity that the biologically active neurotoxic component had prior to being freeze-dried and reconstituted in the cosmetic composition.

The term "lyophilization" is used in this document for a treatment of a solution containing the neurotoxic component of the Botulinum toxin, whereas this solution is frozen and dried until only the solid components of the composition are left over. The freeze-dried product of this treatment is therefore defined in this document as "lyophilisate".

In this document the term "reconstitution" is defined as the process of solubilization of said freeze-dried composition of the neurotoxic component. This can be done by adding the appropriate amount of sterile water, e.g. if all necessary components are already contained in the lyophilisate. Or, if this is not the case, it can be done e.g. by adding a sterile saline-solution alone or if applicable with the addition of components comprising e.g. a pH buffer, excipient, cryoprotectant, preservative, analgesic stabilizer or any combination thereof. The saline of before mentioned "saline-solution" is a salt-solution, more preferably being a sodium-chloride (NaCl) solution, still more preferably being an isotonic sodium-chloride solution (i.e. a sodium-chloride concentration of 0,9%). The solubilization is carried out in such a manner that the final "reconstitution" is directly or indirectly, i.e. for example after dilution, administrable to the patient. Preferably, the neurotoxin is reconstituted in isotonic media. More preferably in isotonic saline. More preferably, said saline is sterile saline.

The terms "preservative" and "preservatives" refer to a substance or a group of substances, respectively, which prevent the growth or survival of microorganisms, insects, bacteria or other contaminating organisms within said composition. Preservatives also prevent said composition from undesired chemical changes. Preservatives which can be used in the scope of this patent are all preservatives of the state of the art known to the skilled person. Examples of preservatives that might be used include, inter alia, e.g. benzylic alcohol, benzoic acid, benzalkonium chloride, calcium propionate, sodium nitrate, sodium nitrite, sulphites (sulfur dioxide, sodium bisulfite, potassium hydrogen sulfite, etc.), disodium EDTA, formaldehyde, glutaraldehyde, diatomaceous earth, ethanol, methyl chloroisothiazolinone, butylated hydroxyanisole and/or butylated hydroxytoluene.

"Stabilizing", stabilizes" or "stabilization" means that the active ingredient, i.e., the neurotoxic component in a reconstituted or aqueous solution cosmetic composition has greater than about 20%, 30%, 40%, 50%, 60%, 70%, 80%, 90%, and up to about 100% of the toxicity that the biologically active neurotoxic component had prior to being incorporated into the cosmetic composition. The activity of the preparation may be determined as described elsewhere herein.

Examples of such stabilizers are gelatin or albumin, preferably of human origin or obtained from a recombinant source. Preferably, the albumin is in a concentration of from about 0.1 to about 10mg per 100 Units botulinum toxin, more preferably the albumin concentration is about 1mg per 100 Units botulinum toxin, wherein the term "about" is to be interpreted as ± 20%. Also preferred are albumin concentrations in the range of 0.1 to about 10mg per 800pg neurotoxic component, wherein an albumin concentration of about 1 mg per 800pg neurotoxic component is preferred. The term "about" is to be interpreted as ± 20% [w/w].

The stabilizers may be modified by chemical means or by recombinant genetics or both.

In a more preferred embodiment of the present invention, the stabilizer may be a nonproteinaceous stabilizing agent comprising hyaluronic acid or polyvinylpyrrolidone or polyethylene glycol or a mixture of two or more thereof. Such composition is considered to be a safer composition possessing remarkable stability.

In a more preferred embodiment of the present invention, the cosmetic composition may comprise the neurotoxic component and a hyaluronic acid or a polyvinylpyrrolidone or a polyethylene glycol, such composition being optionally pH stabilized by a suitable pH buffer, in particular by a sodium acetate buffer, and / or a cryoprotectant polyalcohol.

Whether or not the cosmetic composition comprises, beside the neurotoxin component, additional components such as albumin, hyaluronic acid, a polyvinylpyrrolidone and/or a polyethylene glycol stabilizer, the cosmetic composition retains its potency substantially unchanged for six month, one year, two year, three year and/or four year periods when stored at a temperature between about +8°C. and about -20°C. Additionally, the indicated cosmetic compositions may have a potency or percent recovery of between about 20% and about 100% upon reconstitution.

A cosmetic composition within the scope of the present invention may include the neurotoxic component one or more additional components. Preferably, the cosmetic compositions disclosed herein, has a pH of between about 4 and 7.5 when reconstituted or upon injection, more preferably between about pH 6.8 and pH 7.6 and most preferably between pH 7.4 and pH 7.6. Generally, the cosmetic composition of the present invention comprises neurotoxic component in a quantity of about 6pg to 30 ng.

***Alternative:** the cosmetic composition comprises neurotoxic component in a quantity of about 2 pg to 50 ng. Preferred quantity ranges are in the range of from 2 pg to 200 pg, 200 pg to 400 pg, 400 pg to 600 pg, 600 pg to 800 pg, 800 pg to 1 ng, 1 ng to 1,5 ng, 1,5 ng to 2 ng, 2 ng to 2,5 ng, 2,5 ng to 3 ng, 3 to 3,5 ng, 3,5 to 4 ng, 4 ng to 4,5 ng, and 4,5 to 5 ng.*

Preferably, the neurotoxic component has a biological activity of 50 to 250 LD50 units per ng neurotoxic component, as determined in a mouse LD50 assay. More preferably, the neurotoxic component has a biological activity of about 150 LD50 per ng neurotoxic component.

The cosmetic composition of the present invention may comprise a neurotoxin, and a hyaluronic acid. The hyaluronic acid stabilizes the neurotoxin. The cosmetic compositions disclosed herein may have a pH of between about 4 and 7.5 when reconstituted or upon injection. The hyaluronic acid in the instant cosmetic composition is preferably combined with the instant neurotoxic component in a quantity of 0.1 to 10 mg, especially 1 mg hyaluronic acid per ml in a 200 U/ml botulinum toxin solution. More preferably, the subject solution also contains a 1-100 mM, especially 10 mM sodium acetate buffer.

In another preferred embodiment, the composition may contain a polyalcohol as cryoprotectant. Examples of polyalcohols that might be used include, e.g., inositol, mannitol and other non-reducing alcohols.

In particular those embodiments of the present invention's cosmetic composition not comprising a proteinaceous stabilizer, preferably do not contain trehalose or maltotriose or related sugar or polyhydroxy compounds which are sometimes used as cryoprotectants.

The polyvinylpyrrolidone in the instant cosmetic composition is preferably combined with the instant neurotoxic component in a quantity of 10 to 500 mg, especially 10 mg polyvinylpyrrolidone per ml in a 200 U/ml botulinum toxin solution. More preferably, the subject solution also contains a 1-100 mM, especially 10 mM sodium acetate buffer.

The polyethylene glycol in the instant cosmetic composition is preferably combined with the instant neurotoxic component in a quantity of 10 to 500 mg, especially 100 mg polyethylene glycol per ml in a 200 U/ml botulinum toxin solution. More preferably, the subject solution also contains a 1-100 mM, especially 10 mM sodium acetate buffer. This ratio of components is also applied in case lower concentrations of down to 25U/ml neurotoxic component solution.

Thus, the instant invention encompasses in a more preferred embodiment a neurotoxic component formulated in a cosmetic composition, which contains a hyaluronic acid stabilizer or a polyvinylpyrrolidone stabilizer or a polyethyleneglycol stabilizer or any combination thereof. Additionally, the cosmetic compositionmay contain a sodium acetate buffer system and/or an alcoholic cryoprotectant or both. In a further preferred embodiment the formulation is albumin free, and comprises as a stabilizer hyaluronic acid, polyvinylpyrrolidone (Kollidon^{®}), hydroxyethyl starch or alginate or a mixture of two or more of these. Said preferred composition comprises in addition to the mentioned stabilizers water and at least one polyalcohol, preferably mannitol or sorbitol or mixtures thereof.

### Examples:

### Example 1:

A 43 year old male is treated for glabellar frown lines. He had been treated with the Botulinum toxin complex before and suffered from "frozen face". He defines his treatment objectives as treatment of his moderately severe forehead lines but keeping his dynamic mimic and facial appearance.

100 Units of Neurotoxin are diluted in 2.5 ml unpreserved 0.9% Saline solution.

He is injected at 5 injection points receiving in total 40 Units of Neurotoxin in 5 aliquots corresponding to 8 Units of Neurotoxin each. Said dosing corresponded to the dosing of the earlier treatment with the complex.

One injection on each facial side is applied in the middle part of the corrugator muscles, 1.6 cm above the bony orbital rim on an imaginary line drawn from the midpupillary line.

One injection on each facial side is given in the central part of the corrugator muscles 1.1 cm above the bony orbital rim on an imaginary vertical line from the caruncle.

One injection is given in the procerus muscle at the imaginary crossing of to lines connecting the second injection point and the contralateral caruncle.

Treatment effect is evaluated after 4 days and 14 days and rated by the patients. Patient is highly satisfied either with the treatment effect and his dynamic mimic and facial appearance.

### Example 2:

A 28 year old female is treated for glabellar frown lines. Treatment should be for prophylactic purpose but patient raises concerns that she might have a "frozen face" after treatment.

Patient receives in total 20 units Neurotoxin diluted in 0.5 ml 0.9% unpreserved Saline solution. Neurotoxin is injected in 5 equal aliquots containing 4 Units each, which corresponds to the dosing of the earlier treatment with the complex.

One injection on each facial side is applied in the middle part of the corrugator muscles, 1.5 cm above the bony orbital rim on an imaginary line drawn from the midpupillary line.

One injection on each facial side is given in the central part of the corrugator muscles 1.0 cm above the bony orbital rim on an imaginary vertical line from the caruncle.

One injection is given in the procerus muscle at the imaginary crossing of to lines connecting the second injection point and the contralateral caruncle.

Treatment effect is evaluated after 10 days. Patient is satisfied with the treatment effect. She is not experiencing impairment of her mimic expressions and appearance.

### Example 3:

A 45 year old male is treated for Crows Feet. Patient wants to keep his facial expression especially while he is laughing. He is concerned to loose this ability because of the treatment, as he had experienced after the treatment with the Botulinum toxin complex.

100 Units of Neurotoxin are diluted in 2.5 ml unpreserved 0.9% Saline solution.

Patient receives in total 6 injections, 3 at each facial site and a total dose of 24 Units Neurotoxin, which corresponds to the dosing of the earlier treatment with the complex.

One injection of 0.1 ml is placed 1.1 cm lateral from the orbital bony rim. This correlates with the 09:00/03:00-o'clock position of the orbiculari oculi muscle. Two additional injections of 0.1 ml are placed 1.0 cm above and below the area of the first injection corresponding to the 10:00/02:00 o'clock respectively a 08:00/04:00 o'clock position.

Treatment effect is assessed after 7 days. Patient is highly satisfied with his appearance at rest and while he is laughing.

### Example 4:

A 38 year old female is treated for Crows Feet. Patient likes to reduce her lower lateral canthal rhytides while upper lines should support her positive appearance for example while she is laughing.

100 Units of Neurotoxin are diluted in 2.5 ml unpreserved 0.9% Saline solution.

Patient receives in total 4 injections of 0.08 ml each, which corresponds to the dosing of the earlier treatment with the complex. One injection is placed 1.0 cm lateral from the orbital bony rim. This correlates with the 09:00/03:00-o'clock position of the orbiculari oculi muscle. One additional injection is placed 1.0 cm below the area of the first injection corresponding to the 08:00/04:00 o'clock position.

Treatment effect is assessed after 3 days. Patient is highly satisfied with the result.

### Example 5:

A 46 year old female is treated for Glabellar Lines and her Forehead Lines. Patient is concerned about the treatment while she already experienced "Doll's Face" by previous treatment with Botulinum Toxin.

100 Units of Neurotoxin are diluted in 2.5 ml unpreserved 0.9% Saline solution.

Patient receives 5 injections and a total dose of 30 Units Neurotoxin for the treatment of the Glabellar Area and additional 4 injections with a total dose of 16 Units to treat the forehead area, which corresponds to the dosing of the earlier treatment with the complex.

One injection on each facial side is applied in the middle part of the corrugator muscles, 1.5 cm above the bony orbital rim on an imaginary line drawn from the midpupillary line.

One injection on each facial side is given in the central part of the corrugator muscles 1.0 cm above the bony orbital rim on an imaginary vertical line from the caruncle.

One injection is given in the procerus muscle at the imaginary crossing of to lines connecting the second injection point and the contralateral caruncle.

Frontalis muscle is injected on each facial side on imaginary crossing of vertical midpupillary line and a horizontal line half distance between the brows and the hair. Two additional injections are applied in equal distances on the horizontal line between the first two injections.

Treatment effect is assessed after 14 days. Patients is highly satisfied with the outcome and not longer concerned about a "Doll's Face".

### Example 6:

A 37 year old actress is treated is treated for her Forehead Lines. Her objective for the treatment is to keep the dynamic mimic and her facial expression. She is highly concerned to suffer from a "Frozen -" or "Doll's Face" as she had experienced after the treatment with the Botulinum toxin complex.

Patient receives 4 Injections of 12 Units Neurotoxin in total, which corresponds to the dosing of the earlier treatment with the complex. 3 Units aliquots are applied to the frontalis muscle on imaginary crossing of vertical midpupillary line and a horizontal line half distance between the brows and the hair. Two additional injections are applied in equal distances on the horizontal line between the first two injections.

Treatment effect is assessed 14 days after treatment. Patient is satisfied with the outcome.

### Example 7:

A 36 year old female is treated for Glabellar Lines. Patient is concerned about the treatment while she already experienced "Doll's Face" by previous treatment with Botulinum Toxin.

Patient receives 5 injections and a total dose of 20 Units Neurotoxin for the treatment of the Glabellar Area, which corresponds to the dosing of the earlier treatment with the complex.

One injection on each facial side is applied in the middle part of the corrugator muscles, 1.5 cm above the bony orbital rim on an imaginary line drawn from the midpupillary line.

One injection on each facial side is given in the central part of the corrugator muscles 1.0 cm above the bony orbital rim on an imaginary vertical line from the caruncle.

One injection is given in the procerus muscle at the imaginary crossing of to lines connecting the second injection point and the contralateral caruncle.

Treatment effect is assessed 14 days after treatment. Patient is highly satisfied with the outcome. She does not report any impairment of her facial appearance.

### Example 8:

A 55 year old female is treated for her perioral lines for the first time. Patient is highly concerned about possible impairments on lip movement and lip appearance as she had experienced after the treatment with the Botulinum toxin complex.

Patient is injected with a total dose of 6 units Neurotoxin, which corresponds to the dosing of the earlier treatment with the complex. This dose is applied in 4 equal aliquots injected into 4 lines on the upper lip.

Treatment effect is assessed 10 days after treatment. Patient is satisfied with the treatment result.

## Claims

1. Use of a neurotoxic component of a *Clostridium* botulinum toxin complex for the facial cosmetic treatment of a human or animal susceptible to suffering from doll's face or frozen face.

2. Use as claimed in claim 1, wherein the human or animal have been treated with a botulinum toxin complex and have suffered from doll's face or frozen face as a result of said treatment with the botulinum toxin complex.

3. Use as claimed in claim 1 or 2, wherein said facial cosmetic treatment is the smoothening of facial lines or facial wrinkles of the skin.

4. Use as claimed in any one of the preceding claims, wherein the human or animal have been treated with a botulinum toxin complex to smoothen facial lines or facial wrinkles of the skin.

5. Use as claimed in claim 4, wherein the facial line or facial wrinkle is selected from the group consisting of glabella frown lines, frown lines, worry lines, marionette lines, horizontal forehead line, crow's feet, nose perioral fold, mental ceases, popply chin, and/or platysmal bands.

6. Use as claimed in any one of the preceding claims, wherein the neurotoxic component is intramuscularly or subcutaneously injected.

7. Use as claimed in claim 6, wherein the neurotoxic component is directly injected into the facial line or facial wrinkle of the skin to be smoothed.

8. Use as claimed in claim 6, wherein the neurotoxic component is directly injected into, or in vicinity of, one or two facial muscles or muscles involved in the formation of the line or wrinkle of the skin to be smoothed.

9. Use as claimed in claim 8, wherein the muscles are selected from the group consisting of corrugator supercillii, orbicularis oculi, procerus, venter frontalis of occipitofrontalis, orbital part of orbicularis oculi, nasalis, upper lip: orbicularis oris, lower lip: depressor angulis oris, mentalis and platysma.

10. Use as claimed in any one of the preceding claims, wherein the neurotoxic component is selected from the group consisting of type A, B, C, D, E, F, G, or a mixture of two or more of said types.

11. Use as claimed in any one of the preceding claims, wherein the neurotoxin complex has a molecular weight below 200 kDa.

12. Use of any one of the preceding claims, wherein the neurotoxic component is dissolved in physiological saline solution.

13. Use as claimed in any one of the preceding claims, wherein the amount of the neurotoxin component is from 2 pg to 50 ng.

14. Use of claim 13, wherein the neurotoxic component has a biological activity of 50 to 250 LD₅₀ units per ng neurotoxic component.

15. Neurotoxic component of a *Clostridium* botulinum toxin complex for the treatment of a disease or disorder caused by or associated with hyperactive cholinergic innervation of muscles or exocrine glands in a human or animal susceptible to side effects associated with the treatment of said disease or disorder with a *Clostridium* botulinum toxin complex.

16. Neurotoxic component as claimed in claim 15, wherein the side effects are one or more of the following: pain, soreness or bruising at the injection site, fever (pyrexia), flu-like symptoms, difficulty or pain when swallowing (dysphagia), rash or itching, bruising of soft eyelid tissues, drooping of the upper eyelid, sensitivity of the eyes to light, eye irritation, dry eyes, watering eyes, facial swelling, difficulty closing the eyelid, inflammation of the front of the eye (cornea), dizziness, increased rigidity, stiffness or soreness of muscles, numbness, weakness, drowsiness, nausea, general feeling of being unwell/malaise, viral or ear infections, urinary incontinence, sleepiness, tingling, arm pain, increased rigidity of muscles, increased sweating from areas not treated, hot flushes.

17. Neurotoxic component as claimed in claim 15 or 16, wherein the human or animal have been treated with a botulinum toxin complex and have suffered from one or more of the side effects as defined in claim 16 as a result of said treatment with the botulinum toxin complex.

18. Neurotoxic component as claimed in any one of claims 15 to 17, wherein the neurotoxic component is intramuscularly or subcutaneously injected.

19. Neurotoxic component as claimed in claim 18, wherein the neurotoxic component is directly injected into, or in the vicinity of one or two muscles or one or two exocrine glands.

20. Neurotoxic component as claimed in claim 19, wherein the muscle is ipsilateral splenius, contralateral sternocleidomastoid, ipsilateral sternocleidomastoid, splenius capitis, scalene complex, levator scapulae, postvertebralis, ipsilateral trapezius, levator scapulae, bilateral splenius capitis, upper trapezius, deep postvertebralis, bilateral sternocleidomastoid, scalene complex, submental complex, brachioradialis, biceps brachialis, pronator quadratus pronator teres, flexor carpi radialis, flexor carpi ulnaris, flexor pollicis longus, adductor pollicis, flexor pollicis brevis/opponens, flexor digitorum superficialis, flexor digitorum profundus.

21. Neurotoxic component as claimed in claim 19, wherein the exocrine gland is sweat gland, tear gland, salivary gland and mucosal gland; or gland associated with a disease or condition selected from the group consisting of Frey syndrome, Crocodile Tears syndrome, axillar hyperhidrosis, palmar hyperhidrosis, plantar hyperhidrosis, hyperhidrosis of the head and neck, hyperhidrosis of the body, rhinorrhea, or relative hypersalivation in patients with stroke, Parkinson's disease or Amyotrophic Lateral Sclerosis.

22. Neurotoxic component as claimed in any one of claims 15 to 21, wherein the neurotoxic component is selected from the group consisting of type A, B, C, D, E, F, G, or a mixture of two or more of said types.

23. Neurotoxic component as claimed in any one of claims 15 to 22, wherein the neurotoxin complex has a molecular weight below 200 kDa.

24. Neurotoxic component as claimed in any one of claims 15 to 23, wherein the neurotoxic component is dissolved in physiological saline solution.

25. Neurotoxic component as claimed in any one of claims 15 to 24, wherein the amount of the neurotoxin component is from 2 pg to 50 ng.

26. Neurotoxic component as claimed in claim 25, wherein the neurotoxic component has a biological activity of 50 to 250 LD₅₀ units per ng neurotoxic component.
